# EUROPÄISCHE PATENTSCHRIFT

(11) **EP 2 462 874 B1**
(45) Veröffentlichungstag und Bekanntmachung des Hinweises auf die Patenterteilung: **05.02.2020**
(21) Anmeldenummer: 10015519.1
(22) Anmeldetag: 10.12.2010
(51) Int. Cl.: A61B 17/00

(54) **Medizinisches Instrument für mikroinvasive Anwendungen**
Medical instrument for micro-invasive applications
Instrument médical pour applications micro-invasives

(43) Veröffentlichungstag der Anmeldung: 13.06.2012
(73) Patentinhaber: Carl Freudenberg KG, 69469 Weinheim (DE)
(72) Erfinder: Neumueller, Daniel, 69469 Weinheim (DE); Reibel, Denis, Dr., 67850 Herrlisheim (FR); Grafahrend, Dirk, Dr., 68165 Mannheim (DE)

(56) Entgegenhaltungen:
- WO-A2-2005/016152
- US-A1- 2006 025 815
- US-A1- 2006 100 664

## Beschreibung

### Technisches Gebiet

Die Erfindung betrifft ein medizinisches Instrument gemäß dem Oberbegriff des Patentanspruchs 1.

### Stand der Technik

Ein häufig bei minimal-invasiven Operationen verwendetes medizinisches Instrument ist der Trokar. Der Trokar ist ein Instrument, mit dessen Hilfe in der minimal-invasiven Chirurgie scharf oder stumpf ein Zugang zu einer Körperhöhle (z. B. Bauchraum oder Brustraum) geschaffen und durch ein zylindrisches, tubusartiges Rohr offengehalten wird.

Moderne Trokare sind entweder aus Titan, chirurgischem Stahl oder Kunststoff gefertigt und werden als einmalig oder mehrfach verwendbare Instrumente hergestellt.

In den Trokar kann als Darreichungs- oder Transportvorrichtung ein Tubus mit einem Stößel eingeführt werden, wobei Tubus und Stößel aus Kunststoff gefertigt sein können.

Dabei sitzt ein Stößel in einem Tubus mit einem Innendurchmesser von etwa 0,5 - 12 mm, wobei die Spitze des Stößels die Öffnung des Tubus verschließt. Tubus und Stößel befinden sich zumindest teilweise innerhalb des Trokars und werden durch diesen durch die Bauchdecke zumindest teilweise in den Bauchraum eingeführt. Durch Tubus und Stößel können Materialien in den Bauchraum transportiert werden, welche die Heilung begünstigen.

Ein Operateur kann nach dem Entfernen des Stößels und/ oder des Tubus mit einer Optik durch den Trokar in den Bauchraum schauen oder mit Greif-, Schneide- und anderen Instrumenten innerhalb des Bauchraums minimalinvasiv operieren.

Trokare werden in der Laparoskopie, Thoraskopie und Arthroskopie zur Inspektion von Körper- oder Gelenkhöhlen verwendet.

In der Medizintechnik besteht häufig der Bedarf, biologisch abbaubare Materialien in den menschlichen oder tierischen Körper zu verbringen, um eine Heilung zu begünstigen oder Blutungen zu stillen.

Dabei ist es notwendig, die Materialien zerstörungsfrei an die Ihnen zugedachte Position zu verbringen. Die bisher verwendeten biologisch abbaubaren Materialien weisen eine unzureichende mechanische Stabilität auf. Insbesondere im nassen Zustand sind die bisher verwendeten Materialien unzureichend stabil.

Das Dokument US 2006/0100664 A1 offenbart eine Vorrichtung zur Versiegelung einer vaskulären Punktur umfassend einen Tubus und einen innerhalb des Tubus aufgenommenen Stößel sowie einen Stopfen. Der Stopfen ist dafür vorgesehen in eine Wunde eingebracht zu werden und dort zu verbleiben. Zu diesem Zweck kann der Stopfen einen Kern aufweisen, der aus einem biokompatiblen und/oder bioabsorbierbaren Material, wie beispielsweise einem porösen bioabsorbierbarem Schaum oder einem anderen festen Material bzw. einem bioabsorbierbarem Hydrogel wie Polyethylenglycol geformt ist. Der Kern kann Gelatine enthalten.

### Darstellung der Erfindung

Die dem Anmeldungsgegenstand zugrundeliegende Aufgabe wird durch die im Anspruch 1 angegebenen Merkmale gelöst, weitere Ausführungsformen sind in den Unteransprüchen aufgeführt. Der Erfindung liegt die Aufgabe zugrunde, ein medizinisches Instrument anzugeben, welches derart ausgerüstet ist, dass ein biologisch abbaubares Material zerstörungsfrei durch einen Tubus transportierbar und problemlos positionierbar ist.

Die vorliegende Erfindung löst die zuvor genannte Aufgabe durch ein medizinisches Instrument mit den Merkmalen des Patentanspruchs 1.

Danach umfasst ein medizinisches Instrument einen Tubus und einen innerhalb des Tubus aufgenommenen Stößel, wobei der Stößel relativ zum Tubus beweglich und innerhalb dessen verschiebbar ist und wobei der Stößel ein erstes Ende des Tubus verschließt. Im Tubus ist ein biologisch abbaubares Vlies aufgenommen, wobei das Vlies durch Betätigen des Stößels aus dem Tubus ausbringbar ist. Überraschend ist erkannt worden, dass biologisch abbaubare Vliese eine sehr starke Dehnbarkeit und Stabilität zeigen können und auch durch enge zylindrische Räume innerhalb eines Tubus gepresst werden können. Obwohl der Fachmann erwartet hätte, dass derart filigrane Gebilde wie biologisch abbaubare Vliese beim Schieben durch einen Tubus zerreissen, wurde dieser Weg erfindungsgemäß erfolgreich beschritten. Besonders überraschend wurde herausgefunden, dass biologisch abbaubare Vliese auch im nassen oder feuchten Zustand, insbesondere nach Tränkung mit Körperflüssigkeiten, ausreichend stabil verbleiben. Hierdurch ist ein medizinisches Instrument angegeben, welches derart ausgerüstet ist, dass ein biologisch abbaubares Material zerstörungsfrei durch einen Tubus transportierbar und problemlos positionierbar ist.

Folglich ist die eingangs genannte Aufgabe gelöst.

Das Vlies könnte durch ein Rotationsspinnverfahren hergestellt sein, wobei einige Fasern des Vlieses miteinander verdrillt sind. sind. Beim Rotationsspinnverfahren bilden sich Faserstränge aus miteinander verdrillten Fasern. Hierdurch erhält das Vlies eine besondere Dehnbarkeit und Stabilität. Vliese dieser Art und Verfahren zur Herstellung solcher Vliese sind in den DE 10 2005 048 939 A1, DE 10 2007 011 606 A1, DE 10 2007 044 648 A1, EP 2 042 199 A2 und der DE 10 2010 012 845.7 offenbart.

Das Vlies könnte nach Beaufschlagung mit einer Flüssigkeit oder im nassen Zustand derart stabil sein, dass es vernähbar, verklebbar oder in ähnlicher Weise fixierbar ist. Hierdurch ist das Vlies problemlos innerhalb eines menschlichen oder tierischen Körpers verarbeitbar.

Vor diesem Hintergrund könnte das Vlies im nassen oder feuchten Zustand zumindest um 30 %, bevorzugt zumindest um 70%, seiner Ausgangslänge oder Ausgangsbreite im unbelasteten Zustand zerstörungsfrei dehnbar sein. Hierdurch ist das Vlies problemlos innerhalb eines menschlichen oder tierischen Körpers vernähbar, verklebbar oder in ähnlicher Weise fixierbar. Das Vlies zeigt eine biologische Abbaubarkeit im menschlichen oder tierischen Körper. Hierdurch kann das Vlies auf eine Wunde aufgelegt werden und mit dem menschlichen oder tierischen Körpergewebe problemlos verwachsen oder von diesem abgebaut werden.

Der Stößel könnte als medizinisches Werkzeug, bevorzugt als chirurgische Zange, ausgebildet sein. Hierbei ist vorteilhaft, dass das Werkzeug, welches beispielsweise für operative Eingriffe verwendet wird, gleichzeitig zum Ausbringen des Vlieses eingesetzt werden kann.

Zumindest eine Komponente des Vlieses könnte einen Wirkstoff enthalten oder aus einem Wirkstoff gefertigt sein. Hierdurch können einem Menschen oder einem Tier Wirkstoffe in Faserform zugeführt werden. Es ist denkbar, Vliese zu fertigen, in deren Fasern Wirkstoffe integriert sind.

Zumindest eine Komponente könnte einen Stoff aufweisen, dessen Struktur nach mindestens zwei Minuten Erwärmung bei einer Temperatur von mindestens 50° C zerstört wird. Unter Zerstörung der Struktur wird hierbei auch eine Minderung der spezifischen Wirksamkeit des Stoffes verstanden. Ein solcher Stoff kann als Arzneimittel, insbesondere als Antibiotikum, Enzym, Wachstumsfaktor oder schmerzreduzierendes Mittel ausgestaltet sein. Zumindest eine Komponente könnte ein Antibiotikum enthalten. Antibiotika unterdrücken das Wachstum von Bakterien oder Keimen.

Zumindest eine Komponente könnte ein Enzym enthalten. Enzyme können Stoffwechselvorgänge steuern.

Zumindest eine Komponente könnte einen Wachstumsfaktor enthalten. Wachstumsfaktoren können das Zellwachstum beeinflussen.

Zumindest eine Komponente könnte ein schmerzreduzierendes Mittel enthalten. Hierdurch können die Vliese auf Wunden aufgelegt werden und Wundschmerzen lindern.

Das Vlies kann aus einer oder mehreren Schichten aufgebaut sein. Die Schicht oder die Schichten könnten aus folgenden Polymeren oder Polymermischungen gefertigt sein:
Synthetische biodegradierbare Polymere wie Polylaktide, Polylaktid-co-Glykolid Copolymere, z.B. Resomer RG 502 H, Polylaktid-block-Polyethylenoxide, z.B. Resomer RGP d 5055, Polycaprolactone, Polycaprolacton-block-Polyethylenoxide, Polyanhydride, z.B. Polifeprosan, Polyorthoester, Polydioxanone, Polyphosphoester, z.B. Polylactophate, synthetische biokompatible Polymere bzw. Polymere, die in der Medizin Verwendung finden, wie Polyethylenglykole, Polyethylenoxide, Polyvinylpyrrolidon, Polyvinylalkohole, Polyethylene, Polypropylene, Polyurethane, Polydimethylsiloxane, Polymethylmethacrylate, Polyvinylchloride, Polyethylenterephthalate, Polytetrafluoroethylene, Poly-2-hydroxyethylmethacrylate, natürliche Biopolymere wie Proteine und Peptide, Polysaccharide, Lipide, Nukleinsäuren und speziell Gelatinen, Kollagene, Alginate, Cellulosen, Elastine, Stärken, Chitine, Chitosane, Hyaluronsäure, Dextrane, Schellack, Polymer-Wirkstoff-Konjugate, nämlich an ein biodegradierbares oder biokompatibles Polymer gebundener Wirkstoff oder Additiv, und Copolymere der oben genannten Polymerklassen.

Den Vliesen könnten folgende Wirkstoffe beigemengt sein:
Hier könnten Enzyme, antimikrobielle Agentien, Vitamine, Antioxydantien, Antiinfektiva, Antibiotika, Antiseptika, Antivirale Wirkstoffe, "anti-rejection agents", Analgetika, analgetische Kombinationen, entzündungshemmende Mittel, wundheilungsfördernde Mittel, Hormone, Steroide, Testosteron, Estradiol, Peptide und/oder Peptidsequenzen immobilisierte adhäsionsfördemde Peptidsequenzen, wie Peptidsequenzen und Peptidfragmente von extrazellularen Matrixproteinen, insbesondere Peptide, welche eine oder mehrere der Aminosäuresequenzen RGD-, LDV-, GFOGER-, IKVAV-, SWYGLR-, COMP-, ADAM-, POEM-, YIGSR-, GVKGDKGNPGWPGAP-, cyclo-DfKRG-, KRSR- enthalten, isolierte und/oder genetisch hergestellte Proteine, Polysaccharide, Glycoproteine, Lipoproteine, Aminosäuren, Wachstumsfaktoren, insbesondere aus den Wachstumsfaktorfamilien TGF, (besonders TGF-β), FGF, PDGF, EGF, GMCSF, VEGF, IGF, HGF, IL-1B, IL8 und NG, RNA siRNA, mRNA und/ oder DNA, oder biologische Signalmoleküle, wie z.B. Sonic Hedgelog, Anticancer agents, wie Paclitaxel, Doxorubicin, 1,3-bis-2-chloroethyl-1-nitrosourea BCNU, Camphothecin, lebende Zellen, Opiate, Nikotin, Nitroglycerin, Clonidin, Fentanyl, Scopolamin, Rapamycin, Sirolimus, Gentamicinsulfat, Gentamicincrobefat, Aminosulfonsäuren, Sulfonamidopeptide, peptidanaloge Moleküle auf Basis von D-Aminosäuren, Furanonderivate, Dexamethason, β-Tricalciumphosphat und/oder Hydroxylapatit, insbesondere speziell Hydroxylapatitnanopartikel, in Konzentrationen von 0,000001 - 70% eingesetzt werden.

Ein medizinisches Instrument der hier beschriebenen Art könnte in einem Trokar verwendet werden. Hierzu könnte der Tubus mit Stößel, wobei im Tubus das Vlies aufgenommen ist, in einen Trokar eingeführt werden. Der Trokar selbst kann dann durch eine Körperöffnung eingeführt werden.

Ein medizinisches Instrument der hier beschriebenen Art könnte zum Transportieren eines Vlieses verwendet werden, welches als Hämostasevlies ausgestaltet ist. Vorteilhaft wird das Vlies hierbei zur Blutstillung genutzt.

Ein medizinisches Instrument der hier beschriebenen Art könnte zur Durchführung einer Adhäsionsprophylaxe verwendet werden. Vorteilhaft wird das Vlies hierbei genutzt, um Verwachsungen zwischen Geweben zu verhindern.

Ein medizinisches Instrument der hier beschriebenen Art könnte zum Transportieren eines Vlieses verwendet werden, welches als Trägermaterial für bioaktive Substanzen ausgestaltet ist und in der lokalen Therapie verwendet wird. Vorteilhaft können durch das Vlies bioaktive Substanzen, nämlich Wirkstoffe, Arzneimittel oder ähnliche Stoffe an ihren Zielort innerhalb des menschlichen oder tierischen Körpers verbracht werden.

In dem hier beschriebenen medizinischen Instrument, insbesondere in einem Trokar, können Vliese eingesetzt werden, die durch die DE 10 2005 048 939 A1, DE 10 2007 011 606 A1, DE 10 2007 044 648 A1, EP 2 042 199 A2 und der DE 10 2010 012 845.7 offenbart sind.

Es gibt nun verschiedene Möglichkeiten, die Lehre der vorliegenden Erfindung in vorteilhafter Weise auszugestalten und weiter zu bilden. Dazu ist einerseits auf die nachgeordneten Ansprüche, andererseits auf die nachfolgende Erläuterung bevorzugter Ausführungsbeispiele des erfindungsgemäßen medizinischen Instruments bzw. des Vlieses zu verweisen.

In Verbindung mit der Erläuterung der bevorzugten Ausführungsbeispiele werden auch im Allgemeinen bevorzugte Ausgestaltungen und Weiterbildungen der Lehre erläutert.

### Kurzbeschreibung der Zeichnung

In der Zeichnung zeigen
- Fig. 1: eine Schnittansicht eines Trokars, der in eine Bauchdecke eingeführt ist, wobei das Vlies innerhalb eines Tubus positioniert ist, der im Trokar aufgenommen ist,
- Fig. 2: eine Schnittansicht des Trokars gemäß Fig. 1, der in eine Bauchdecke eingeführt ist, wobei das Vlies teilweise aus dem Tubus gefördert ist, und
- Fig. 3: eine Schnittansicht des Trokars gemäß Fig. 1 und 2, der in eine Bauchdecke eingeführt ist, wobei das Vlies vollständig aus dem Tubus gefördert ist.

### Ausführung der Erfindung

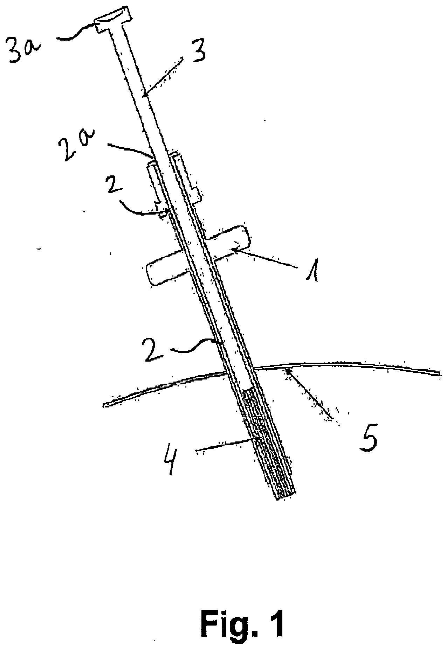

Fig. 1 zeigt einen Trokar 1, in dem ein medizinisches Instrument aufgenommen ist. Fig. 1 zeigt ein medizinisches Instrument, umfassend einen Tubus 2 und einen innerhalb des Tubus 2 aufgenommenen Stößel 3, wobei der Stößel 3 relativ zum Tubus 2 beweglich und innerhalb dessen verschiebbar ist und wobei der Stößel 3 ein erstes Ende 2a des Tubus 2 verschließt. Das erste Ende 2a kann auch durch einen hohlen Stößel 3 zumindest teilweise verschlossen werden.

Im Tubus 2 ist ein biologisch abbaubares Vlies 4 aufgenommen, wobei das Vlies 4 gefaltet oder aufgerollt vorliegen kann und wobei das Vlies 4 durch Betätigen des Stößels 3 aus dem Tubus 2 ausbringbar ist.

Das Vlies 4 ist durch ein Rotationsspinnverfahren hergestellt, wobei einige Fasern des Vlieses 4 miteinander verdrillt sind. Vliese 4 dieser Art sind in den DE 10 2005 048 939 A1, DE 10 2007 011 606 A1, DE 10 2007 044 648 A1, EP 2 042 199 A2 und der DE 10 2010 012 845.7 offenbart.

Das im Tubus 2 aufgenommene Vlies 4 ist im menschlichen oder tierischen Körper biologisch abbaubar.

Das Vlies 4 ist nach Beaufschlagung mit einer Flüssigkeit oder im nassen Zustand derart stabil, dass es vernähbar, verklebbar oder in ähnlicher Weise fixierbar ist.

Das Vlies 4 ist im nassen oder feuchten Zustand zumindest um 30 %, bevorzugt zumindest um 70%, seiner Ausgangslänge oder Ausgangsbreite im unbelasteten Zustand zerstörungsfrei dehnbar.

Der Stößel 3 ist konzentrisch Im Tubus 2 aufgenommen und weist eine Betätigungskuppe 3a auf, auf der ein Daumen oder Finger zur Betätigung des Stößels 3 aufgelegt werden kann.

Der Tubus 2 wiederum ist in einem Trokar 1 konzentrisch aufgenommen, wobei das Vlies 4 innerhalb des Tubus 2 aufgenommen ist und wobei der Stößel 3 innerhalb des Tubus 2 verschiebbar ist. Der Trokar 1 ist durch eine Bauchdecke 5 eines menschlichen Körpers gesteckt.

In Fig. 1 ist das Vlies 4 noch überwiegend Innerhalb des Tubus 2 bzw. innerhalb des Trokars 1 aufgenommen.

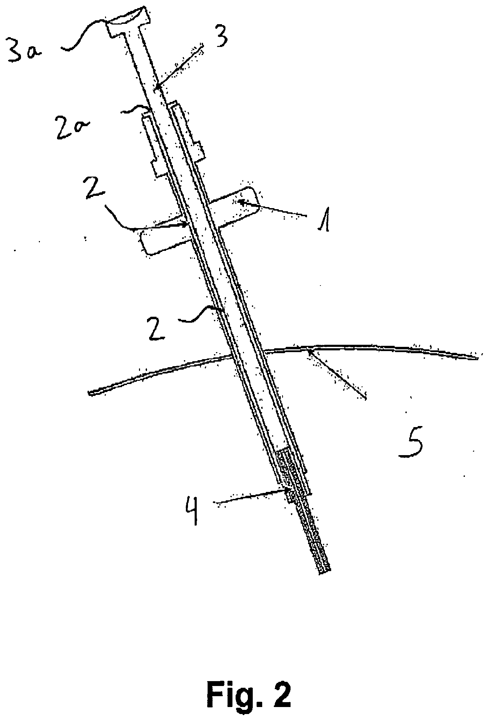

Fig. 2 zeigt einen Betriebszustand, in welchem der Stößel 3 das Vlies 4 mehr als zur Hälfte aus dem Tubus 2 bzw. dem Trokar 1 herausgeschoben hat.

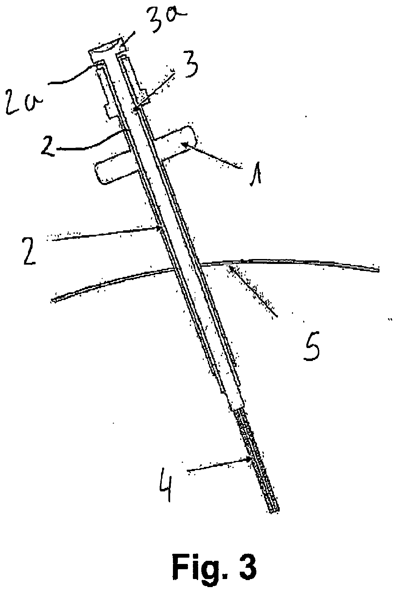

Fig. 3 zeigt einen Betriebszustand, in welchem der Stößel 3 das Vlies 4 vollständig aus dem Tubus 2 bzw. dem Trokar 1 herausgeschoben hat.

Das Vlies 4 kann nun entfaltet oder entrollt und auf ein Organ oder auf einen Knochen aufgelegt werden.

Nachfolgend sind konkrete Ausführungsbeispiele des medizinischen Instruments in Kombination mit verschiedenen Varianten des Vlieses 4 dargestellt.

Die nachfolgend konkret dargestellten Vliese 4 zeichnen sich durch die zuvor beschriebene Stabilität und Dehnbarkeit im nassen Zustand aus.

### Ausführungsbeispiel 1:

Das in den Fig. 1 bis 3 schematisch dargestellte medizinische Instrument besteht konkret aus einem Tubus 2 mit einem Außendurchmesser von 9,8 mm, einem Innendurchmesser von 8 mm und einer Länge von 210 mm. Das medizinische Instrument weist einen Stößel 3 mit einem Durchmesser von 7,95 mm und einer Länge von 215 mm auf. Ein Vlies 4 mit einer Fläche von 50 mm x 50 mm und einem Flächengewicht von 150 g/m² ist im Tubus 2 aufgenommen. Der Tubus 2 und der Stößel 3 bestehen aus Polypropylen (PP).

Ein Bestandteil des medizinischen Instruments ist ein geeignetes Vlies 4, welches als dreidimensionale Struktur zum Verschluss von inneren Wunden oder der Auffüllung von Defekten eingesetzt wird. Dieses Vlies 4 wird durch ein Rotationsspinnverfahren wie folgt hergestellt:
Zur Herstellung des Vlieses 4 wird zunächst eine 20%ige Gelatine-Lösung hergestellt. Zur Verwendung kommt eine Gelatine des Typs A PIGSKIN der Firma GELITA AG. Die Gelatine wird in Wasser eingerührt. Diese Gelatine-Lösung verbleibt zum Quellen für eine Stunde in Ruhe. Anschließend wird die Gelatine-Lösung bei 60° C in einem Ultraschallbad gelöst und dann etwa zwei Stunden auf einer Temperatur von 80-85° C gehalten.

Die auf 80-85° C temperierte Gelatine-Lösung wird mittels einer Schlauchpumpe in ein Behältnis gemäß der DE 10 2005 048 939 A1 geführt. Das Behältnis hat eine Temperatur von ca. 120° C und dreht mit einer Drehzahl von 4500 U/min. Durch die Zentripetalkraft wird das Faserrohmaterial aus den am Behältnis befindlichen Ausnehmungen gepresst und zu Fasern gesponnen. Die Fasern werden durch eine Absaugeinrichtung, die sich unterhalb des Behältnisses befindet, verstreckt

Es wird ein wirksames, bioresorbierbares Vlies 4 aus Gelatine mit einem durchschnittlichen Faserdurchmesser von ca. 12 µm erhalten. Dieses Gelatine-Vlies wird entweder während des Spinnprozesses oder anschließend vernetzt. Eine anschließende Vernetzung kann z.B. über eine Behandlung mit Aldehyden, wie beispielsweise Formaldehyd oder Glutaraldehyd erfolgen. Bei dieser Behandlung wird das Vlies 4 über Nacht zusammen mit einer Schale mit Formaldehyd-Lösung (Sigma-Aldrich, Bestellnummer F8775) in einem Vakuumtrockenschrank gelagert. Nach 24 h wird die Formaldehydlösung entfernt. Der Trockenschrank wird für mindestens 72 h evakuiert und dann belüftet. Ein auf diese Weise behandeltes Gelatine-Vlies ist über einen Zeitraum von mehreren Tagen bis Wochen (vorzugsweise mehr als 4 Wochen) in einem PBS-Puffer stabil. PBS bezeichnet "Phosphate Buffered Saline" (Sigma-Aldrich P5368-10PAK). Hierbei handelt es sich um ein physiologisches Puffermedium mit einem pH-Wert von 7,4, welches als einfachstes Model für Körperflüssigkeiten genutzt wird.

### Ausführungsbeispiel 2:

Das In den Fig. 1 bis 3 schematisch gezeigte medizinische Instrument besteht aus einem Tubus 2 mit einem Außendurchmesser von 9,8 mm, einem Innendurchmesser von 8 mm und einer Länge von 210 mm. Es umfasst einen Stößel 3 mit einem Durchmesser von 7,95 mm und einer Länge von 215 mm. Ein Vlies 4 der Fläche 50 mm x 50 mm mit einem Flächengewicht von 150 g/m² ist im Tubus 2 aufgenommen. Der Tubus 2 und der Stößel 3 bestehen aus Polymethylmethacrylat (PMMA).

Das Vlies 4 weist ein Antibiotikum auf und wird durch ein Rotationsspinnverfahren wie folgt hergestellt:
Zur Herstellung des Vlieses 4 wird zunächst eine 20%ige Gelatine-Lösung hergestellt. Zur Verwendung kommt eine Gelatine des Typs A PIGSKIN gemäß Ausführungsbeispiel 1. Die Gelatine wird in Wasser eingerührt. Diese Gelatine-Lösung verbleibt zum Quellen für eine Stunde in Ruhe. Anschließend wird die Gelatine-Lösung bei 60° C in einem Ultraschallbad gelöst und dann etwa zwei Stunden auf einer Temperatur von 80-85° C gehalten.

Die auf 80-85° C temperierte Gelatine-Lösung wird mittels einer Schlauchpumpe in das Behältnis gemäß der DE 10 2005 048 939 A1 geführt. Kurz vor dem Eintritt der Gelatine-Lösung in die Ausnehmungen wird der Gelatine-Lösung eine Ampulle Gentamicin-Lösung (GENTAMICIN 40 der Firma HEXAL AG) beigemengt. Das Behältnis hat eine Temperatur von ca. 120°C und dreht mit einer Drehzahl von 4500 U/min. Durch die Zentripetalkraft wird das Faserrohmaterial aus den am Behältnis befindlichen Ausnehmungen gepresst und zu Fasern gesponnen. Die Fasern werden durch eine Absaugeinrichtung, die sich unterhalb des Behältnisses befindet, verstreckt. Nach Vernetzung der Gelatine wird ein Vlies 4 mit einem eingeschlossenen Antibiotikum erhalten, welches antimikrobiotisch wirkt und zugleich bioresorbierbar ist.

Es wird ein antimikrobiell wirksames, bioresorbierbares Vlies 4 aus Gelatine mit einem durchschnittlichen Faserdurchmesser von 12 µm erhalten. Das Gelatine-Vlies wird entweder während des Spinnprozesses oder anschließend vernetzt. Eine anschließende Vernetzung kann z.B. über eine Dehydrothermalbehandlung erfolgen. Bei dieser Behandlung wird das Vlies 4 über Nacht in einem Vakuumtrockenschrank behandelt. Der Trockenschrank wird hierzu zuerst evakuiert und dann auf 140° C aufgeheizt. Die auf diese Weise stabilisierten Fasern quellen, lösen sich jedoch nicht sofort in wässriger Umgebung auf. Ein auf diese Weise behandeltes Gelatine-Vlies ist über einen Zeitraum von mehreren Stunden bis Tagen (vorzugsweise mehr als 2 Stunden) wasserstabil.

### Ausführungsbeispiel 3:

Das in den Fig. 1 bis 3 schematisch dargestellte medizinische Instrument besteht aus einem Tubus 2 mit einem Außendurchmesser von 9,8 mm, einem Innendurchmesser von 8 mm und einer Länge von 210 mm. Es umfasst einen Stößel 3 mit einem Durchmesser von 7,95 mm und einer Länge von 215 mm, sowie ein Vlies 4 mit einer Fläche von 50 mm x 50 mm und einem Flächengewicht von 150 g/m². Der Tubus 2 und der Stößel 3 bestehen aus Polyetherketon (PEK).

Das Vlies 4 besteht aus Gelatine und Hydroxylapatit mit einem Antibiotikum und wird durch ein Rotationsspinnverfahren wie folgt hergestellt:
Zur Herstellung des Vlieses 4 wird zunächst eine 20%ige Gelatine-Lösung hergestellt. Zur Verwendung kommt eine Gelatine des Typs A PIGSKIN gemäß Ausführungsbeispiel 1. Die Gelatine wird in Wasser eingerührt. Diese Gelatine-Lösung verbleibt zum Quellen für eine Stunde in Ruhe. Anschließend wird die Gelatine-Lösung bei 60° C in einem Ultraschallbad gelöst, es werden 2,5% nanopartikulärer Hydroxylapatit (Sigma-Aldrich, Bestellnummer: 677418) zugegeben, die Lösung eine halbe Stunde mit Ultraschall behandelt und dann etwa zwei Stunden auf einer Temperatur von 80-85° C gehalten.

Die auf 80-85° C temperierte Gelatine-Lösung wird mittels einer Schlauchpumpe in das Behältnis gemäß der DE 10 2005 048 939 A1 geführt. Das Behältnis hat eine Temperatur von ca. 120°C und dreht mit einer Drehzahl von 4500 U/min. Durch die Zentripetalkraft wird das Faserrohmaterial aus den am Behältnis befindlichen Ausnehmungen gepresst und zu Fasern gesponnen. Die Fasern werden durch eine Absaugeinrichtung, die sich unterhalb des Behältnisses befindet, verstreckt. Die Vernetzung wird nach einem der in den Ausführungsbeispielen 1 und 2 beschriebenen Verfahren durchgeführt. Nach Vernetzung der Gelatine wird das Vlies 4 mit einer Lösung von Gentamicin besprüht und danach getrocknet

Hinsichtlich weiterer vorteilhafter Ausgestaltungen und Weiterbildungen der erfindungsgemäßen Lehre wird einerseits auf den allgemeinen Teil der Beschreibung und andererseits auf die beigefügten Patentansprüche verwiesen.

## Patentansprüche

1. Medizinisches Instrument, umfassend einen Tubus (2) und einen innerhalb des Tubus (2) aufgenommenen Stößel (3), wobei der Stößel (3) relativ zum Tubus (2) beweglich und innerhalb dessen verschiebbar ist und wobei der Stößel (3) ein erstes Ende (2a) des Tubus (2) verschließt,
**dadurch gekennzeichnet, dass** im Tubus (2) ein biologisch abbaubares, durch ein Rotationsspinnverfahren hergestelltes Vlies (4) aufgenommen ist, wobei das Vlies (4) durch Betätigen des Stößels (3) aus dem Tubus (2) ausbringbar ist

2. Instrument nach Anspruch 1, **dadurch gekennzeichnet, dass** das Vlies (4) nach Beaufschlagung mit einer Flüssigkeit oder im nassen Zustand derart stabil ist, dass es vernähbar, verklebbar oder in ähnlicher Weise fixierbar ist.

3. Instrument nach einem der Ansprüche 1 bis 2, **dadurch gekennzeichnet, dass** das Vlies (4) im nassen oder feuchten Zustand zumindest um 30 %, bevorzugt zumindest um 70%, seiner Ausgangslänge oder Ausgangsbreite im unbelasteten Zustand zerstörungsfrei dehnbar ist.

4. Instrument nach einem der Ansprüche 1 bis 3, **dadurch gekennzeichnet, dass** der Stößel (3) als medizinisches Werkzeug, bevorzugt als chirurgische Zange, ausgebildet ist.

5. Verwendung eines medizinischen Instruments nach einem der voranstehenden Ansprüche in einem Trokar (1).

6. Verwendung eines medizinischen Instruments nach einem der Ansprüche 1 bis 4 zum Transportieren eines Vlieses (4), welches als Hämostasevlies ausgestaltet ist.

7. Verwendung eines medizinischen Instruments nach einem der Ansprüche 1 bis 4 zur Durchführung einer Adhäsionsprophylaxe.

8. Verwendung eines medizinischen Instruments nach einem der Ansprüche 1 bis 4 zum Transportieren eines Vlieses (4), welches als Trägermaterial für bioaktive Substanzen ausgestaltet ist und in der lokalen Therapie verwendet wird.

## Claims

1. Medical instrument, comprising a tube (2) and a plunger (3) received inside the tube (2), wherein the plunger (3) is movable relative to the tube (2) and slidable inside the latter, and wherein the plunger (3) closes a first end (2a) of the tube (2), **characterized in that** a biodegradable nonwoven (4) produced by a rotary spinning process is received in the tube (2), wherein the nonwoven (4) can be expelled from the tube (2) by actuation of the plunger (3).

2. Instrument according to Claim 1, **characterized in that** the nonwoven (4), after exposure to a liquid or in the wet state, is stable in such a way that it can be sewn, glued or similarly fixed.

3. Instrument according to one of Claims 1 to 2, **characterized in that** the nonwoven (4) in the wet or moist state is stretchable, without damage, at least by 30%, preferably at least by 70%, of its original length or original width in the unloaded state.

4. Instrument according to one of Claims 1 to 3, **characterized in that** the plunger (3) is configured as a medical instrument, preferably as surgical forceps.

5. Use of a medical instrument according to one of the preceding claims in a trocar (1).

6. Use of a medical instrument according to one of Claims 1 to 4 for transporting a nonwoven (4) which is configured as a haemostasis nonwoven.

7. Use of a medical instrument according to one of Claims 1 to 4 for performing adhesion prevention.

8. Use of a medical instrument according to one of Claims 1 to 4 for transporting a nonwoven (4) which is configured as a carrier material for bioactive substances and is used in local therapy.

## Revendications

1. Instrument médical comprenant un tube (2) et une tige poussoir (3) reçue à l'intérieur du tube (2), la tige poussoir (3) étant mobile par rapport au tube (2) et pouvant être déplacée à l'intérieur de celui-ci, la tige poussoir (3) fermant une première extrémité (2a) du tube (2),
**caractérisé en ce qu'**un non-tissé (4) biodégradable fabriqué par un procédé de filage rotatif est reçu dans le tube (2), le non-tissé (4) pouvant être extrait hors du tube (2) par actionnement de la tige poussoir (3).

2. Instrument selon la revendication 1, **caractérisé en ce que** le non-tissé (4), après avoir été sollicité avec un liquide, ou à l'état mouillé, est suffisamment stable pour pouvoir être cousu, collé ou fixé de manière analogue.

3. Instrument selon l'une quelconque des revendications 1 et 2, **caractérisé en ce que** le non-tissé (4), à l'état mouillé ou humide, peut être étiré sans destruction à au moins 30 %, de préférence à au moins 70 % de sa longueur de départ ou de sa largeur de départ dans l'état non sollicité.

4. Instrument selon l'une quelconque des revendications 1 à 3, **caractérisé en ce que** la tige poussoir (3) est réalisée sous forme d'outil médical, de préférence sous forme de pince chirurgicale.

5. Utilisation d'un instrument médical selon l'une quelconque des revendications précédentes dans un trocart (1).

6. Utilisation d'un instrument médical selon l'une quelconque des revendications 1 à 4 pour le transport d'un non-tissé (4) qui est configuré sous forme de non-tissé hémostatique.

7. Utilisation d'un instrument médical selon l'une quelconque des revendications 1 à 4 pour mettre en œuvre une prophylaxie d'adhérence.

8. Utilisation d'un instrument médical selon l'une quelconque des revendications 1 à 4 pour le transport d'un non-tissé (4) qui est configuré sous forme de matériau support pour des substances bioactives et qui est utilisé en thérapie locale.
